# EUROPEAN PATENT SPECIFICATION

(11) **EP 0 276 049 B1**
(45) Date of publication and mention of the grant of the patent: **17.03.1993**
(21) Application number: 88300059.8
(22) Date of filing: 06.01.1988
(51) Int. Cl.: C07C 51/12, C07C 53/08, C07C 67/36, C07C 69/14, B01J 31/24

(54) **The production of carboxylic acids and esters thereof**
Herstellung von Carbonsäuren und deren Estern
Production d'acides carboxyliques et de leurs esters

(30) Priority: 08.01.1987 GB 8700402
(43) Date of publication of application: 27.07.1988
(73) Proprietor: BP Chemicals Limited, London, SW1W 0SU (GB); THE UNIVERSITY OF SOUTHAMPTON, Southampton, Hampshire SO9 5NH (GB)
(72) Inventor: Evans, John, Romsey Hampshire, SO51 7JY (GB); Scruton, Steven Leonard, Boreham Wood Hertfordshire, WD6 1UY (GB)
(74) Representative: Fawcett, Richard Fennelly

(56) References cited:
- GB-A- 1 342 876
- GB-A- 1 342 877
- US-A- 1 946 258
- US-A- 3 907 852

## Description

The present invention relates in general to the production of carboxylic acids and/or esters thereof and in particular to a process for the production from an alcohol having n carbon atoms of a carboxylic acid having n + 1 carbon atoms or an ester of the alcohol having n carbon atoms with the acid by reacting the alcohol at elevated temperature with carbon monoxide in the presence as catalyst of a noble metal component and a promoter substance which is halogen or a halogen compound.

The carboxylation of alcohols to carboxylic acids and/or esters thereof in the presence of a noble metal component as catalyst and a halide promoter has been known for some considerable time, see for example GB-A-1185453, GB-A-1233121, GB-A-1233122, GB-A-1234641 and GB-A-1234642. Although operation of the process in homogenous media is favoured in the aforesaid patent disclosures, there is also provision for heterogeneous catalysis either in the liquid phase or the vapour phase. For heterogeneous operation the catalysts are dispersed either on an inert support material such as alundum, activated carbon, clays, alumina, silica-alumina and ceramics, or a porous solid carrier, such as a zeolite, a solid foam, a ceramic honeycomb or a porous organic polymer. It is apparent from the prior art that metal oxides are not the preferred support materials. No attempt is made to immobilise the active catalytic component(s) on the support materials, thereby minimising leaching of the catalyst component(s).

Since the early disclosures, attempts to immobilise the catalytic component(s) on polymer substrates by reaction of appropriate functional groups attached to the polymer backbone chain with the noble metal component have been disclosed. Although such catalysts may be effective for liquid phase operation and enjoy the advantage of relatively easy separation and recovery from the liquid reaction medium, they suffer from the disadvantage that they generally undergo thermal degradation at elevated temperatures, thereby rendering them unsuitable for vapour phase operation.

A need therefore arises for a heterogeneous catalyst wherein the catalyst component(s) is (are) immobilised on an inert support, useful for both liquid phase and vapour phase operation of the process.

We have now found that catalysts comprising a noble metal component bonded to an inorganic oxide having surface hydroxyl groups through the intermediacy of a silicon compound which is condensible with the hydroxyl groups of the inorganic oxide and has either (i) nitrogen or phosphorus-containing functional groups capable of coordinating with the noble metal component or (ii) a functional group capable of reacting further with a nitrogen- or phosphorus-containing functional group capable of coordinating with the noble metal component thereby to bond the nitrogen- or phosphorus-containing functional group to the silicon compound satisfy the aforesaid criteria. Although the same or similar compounds have been disclosed for use as catalysts in, for example, hydrogenation, hydroformylation, oxidation and dimerisation processes, their use as alcohols carbonylation catalysts is in our experience novel.

US patent number 3907852 describes heterogenous silyhydrocarbyl phosphine transition metal complex catalysts and their use in liquid phase carbonylation processes.

Accordingly, the present invention provides a process for the production from an alcohol having n carbon atoms, wherein n is a number from 1 to 20, of a carboxylic acid having n + 1 carbon atoms and/or an ester of the alcohol having n carbon atoms with the carboxylic acid by reacting the alcohol at a temperature of at least 50°C with carbon monoxide in the presence of a noble metal-containing heterogeneous catalyst and a halogen or halogen compound promoter characterised in that the process comprises the steps of (a) vaporising the alcohol and (b) contacting the vapour of the alcohol with the carbon monoxide and the catalyst which comprises a noble metal component bonded to an inorganic oxide having surface hydroxyl groups through the intermediacy of a silicon compound which is condensible with the hydroxyl groups of the inorganic oxide and has either (i) at least one nitrogen-containing functional group capable of coordinating with the noble metal component or (ii) a functional group capable of reacting further with a nitrogen-containing functional group capable of coordinating with the noble metal component thereby to bond the nitrogen-containing functional group to the silicon compound.

The term "noble metal" throughout this specification is used to mean rhodium, iridium, platinum, palladium, osmium and ruthenium. Of the noble metals, rhodium and iridium are preferred and rhodium is more preferred.

As regards the catalyst, the inorganic oxide may be any inorganic oxide having surface hydroxyl groups. Examples of suitable inorganic oxides include silica, alumina and the rare earth metal oxides, such as ceria. Preferred inorganic oxides include silica and alumina.

The silicon compound must contain a moiety condensible with the hydroxyl groups of the inorganic oxide. Suitably this moiety may be a -Y group wherein Y is an -OR group in which R is an alkyl group, suitably a lower alkyl group, for example a methyl or an ethyl group or Y is an ester group or a halide group. The silicon compound may in alternative (i) also contain a moiety having a nitrogen or phosphorus-containing functional group capable of coordinating with the noble metal component. Preferably the nitrogen or phosphorus-containing functional group is one capable of quaternisation. Suitably the silicon compound (i) may have the formula: 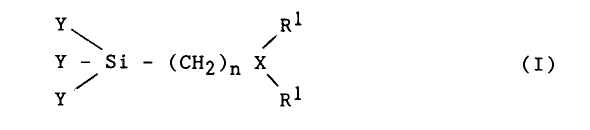
wherein the groups Y are independently -OR groups in which the groups R are alkyl groups, preferably C₁ to C₄ alkyl groups, or the groups Y are independently ester groups or halide groups,
n is a number in the range from 1 to 6,
X is either nitrogen or phosphorus, and
the groups R¹ are independently either hydrogen, alkyl or together form a ring which may be either a carbocyclic or a heterocyclic nitrogen or phosphorus-containing ring. Examples of suitable silicon compounds (i) which may be used in the production of catalysts useful in the performance of the invention include
(A) 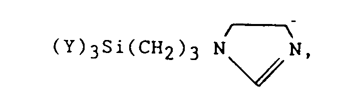
(B) 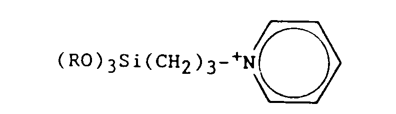 Cl⁻, and
(C) (RO)₃Si(CH₂)₃NH₂.

Alternatively, the silicon compound which is condensible with the hydroxyl groups of the inorganic oxide may contain a functional group capable of reacting further with a nitrogen- or phosphorus-containing functional group capable of coordinating with the noble metal component thereby to bond the nitrogen- or phosphorus-containing functional group to the silicon compound. Such a functional group may be for example a halide group, for example a chloride group, which is capable of reacting further with, for example NR₃ or PR₃, wherein R is independently hydrogen or alkyl, or together form a ring, thereby to bond the NR₃ or PR₃ to the silicon compound. An example of a suitable silicon compound is (MeO)₃Si(CH₂)₃Cl which is reactable with pyridine for example after condensing with the inorganic oxide.

A preferred silicon compound is the compound (A). The noble metal component coordinated with the silicon compound may suitably take the form of an oxide, salt or coordination compound thereof.

The catalyst (i) may suitably be prepared by the steps comprising:
in a first step reacting an inorganic oxide having surface hydroxyl groups with a silicon compound having a first moiety which is condensible with hydroxyl groups and a second moiety which is a nitrogen or phosphorus-containing functional group capable of coordinating a noble metal component under conditions whereby the hydroxyl groups of the inorganic oxide react with the first moiety thereby to bond the silicon compound to the inorganic oxide, and
in a second step reacting the second moiety of the product of the first step with a noble metal component, thereby to coordinate the noble metal component to the silicon compound.

In a modification of this method the noble metal component may be reacted with the second moiety of the silicon compound before this latter compound is reacted via the first moiety with the inorganic oxide.

Preferably as many of the surface hydroxyl groups of the inorganic oxide as possible are reacted with the silicon compound.

We believe, although we do not wish to be bound in any way by our belief, that under the conditions of the process, the nitrogen or phosphorus-containing functional group can be quaternised and the noble metal component is simultaneously ionised. Accordingly, the functional group may be added as such or, in a preferred embodiment of the invention it may be added in a pre-quaternised form, which may suitably be prepared by carrying out the first step of the process as hereinbefore described, in a second step reacting the product of the first step with a quarternising agent, suitably an alkyl iodide, thereby to quaternise the nitrogen or phosphorus-containing functional group and in a third step reacting the product of the second step with a noble metal halocarbonyl complex, thereby to form a noble metal halocarbonyl complex counter anion.

Alternatively, the catalyst (ii) may suitably be prepared by the steps comprising:-
in a first step reacting an inorganic oxide having surface hydroxyl groups with a silicon compound having a first moiety which is condensible with hydroxyl groups and a second moiety which is a functional group capable of reacting further with a nitrogen- or phosphorus-containing compound capable of coordinating with a noble metal component under conditions whereby the hydroxyl groups of the inorganic oxide react with the first moiety thereby to bond the silicon compound to the inorganic oxide,
in a second step reacting the second moiety with a nitrogen- or phosphorus-containing compound under conditions whereby the nitrogen- or phosphorus-containing compound is bonded to the silicon compound, and
in a third step reacting the product of the second step with a noble metal component under conditions whereby the noble metal component is coordinatively bonded to the silicon compound.

As regards the alcohol used as feedstock, this may suitably be an aliphatic alcohol having from 1 to 20 carbon atoms including methanol, ethanol, propanol and isopropanol, the butanols, pentanols and hexanols, and also the higher alcohols such as the decanols, including isomeric forms. Preferably methanol is used as the feedstock to produce acetic acid and/or methyl acetate. The methanol may be essentially pure or may contain impurities commonly associated with commercial grades, for example water and ethanol. Polyhydric alcohols may be employed, as may also aromatic hydroxyl-containing compounds, for example phenol.

Carbon monoxide is readily available on a commercial scale and may contain impurities commonly associated therewith, for example methane, nitrogen and hydrogen, but should be substantially free of oxygen.

The promoter is a halogen or a halogen compound which may be for example a hydrogen halide, an alkyl or aryl halide, a metal halide or an ammonium, phosphonium, arsonium or stibonium halide, which may be the same or different from any halogen component already present in the catalytic component. Those containing iodine are preferred. Preferably the promoter is an alkyl iodide, for example methyl iodide.

The process is operated in the vapour phase.

The process may be operated batchwise or continuously, preferably continuously employing the catalyst in the form of either a fixed or a fluidised bed.

The process is operated at elevated temperature, suitably in the range from 50 to 300, preferably from 100 to 250°C, and a pressure suitably in the range from 1 to 100, preferably from 5 to 50 barg. The Liquid Hourly Space Velocity (LHSV) for vapour or liquid, fixed or fluidised bed operation may suitably be in the range from 0.1 to 20.0, the preferred value within this range being dependent on the temperature and pressure. The molar ratio of carbon monoxide to liquid feed may suitably be in the range from 0.1:1 to 20:1, preferably from 1:1 to 10:1.

Further details regarding reactants, modes of operations and reaction conditions may be found in the aforesaid GB patent publications, the contents of which are incorporated herein by reference.

The invention will now be further illustrated by reference to the following Examples.

### CATALYST PREPARATION

### Catalyst A (alternative (i))

(a) Silica (FK 700;5 g) (dried under vacuo for 48 h at 140°C prior to use) was refluxed with [Et0]₃Si(CH₂)₃N₂C₃H₅ (5g) in toluene (50 ml) and from this mixture was azeotroped toluene containing ethanol using a Dean and Stark apparatus. After 16 h the resulting yellow silica was filtered off and washed with petroleum ether (3 x 30 ml). The silica was transferred to a Soxhlet apparatus and extracted with diethyl ether for 16 h.
(b) Silica obtained in (a) above (5g) was refluxed with methyl iodide (5g) in toluene (30 ml). After 16 hours the resulting bright yellow solid was removed by filtration and washed with petroleum ether (3 x 30 ml). The silica was transferred to a Soxhlet apparatus and extracted with carbon tetrachloride for 16 h. The silica was dried under vacuo.
(c) Rhodium carbonyl chloride [Rh(C0)₂Cl]₂ (0.02 g) dissolved in methanol (10 ml) was added to silica obtained in (b) above (1.8 g). The reaction mixture was stirred for 5 minutes and the resulting brown silica removed by filtration. Its rhodium content was found by analysis to be 1.29% b.w. of dried material.

### Catalyst B (alternative (i))

Rhodium carbonyl chloride, [Rh(C0)₂Cl]₂, (0.02 g) dissolved in methanol (10 ml) was added to silica (FK 700; 1.8g) which had previously been dried in vacuo at 140°C. After stirring for 5 minutes the solvent was removed on a rotary evaporator.

### Catalyst C (alternative (ii))

(a) Gamma-alumina (CONDEA 200 (RTM): 5g) (dried under vacuo for 48h at 140°C prior to use) was refluxed with (MeO)₃Si(CH₂)₃Cl (5g) in toluene (50 ml) and from this mixture was azeotroped toluene and methanol using a Dean and Stark apparatus. After 16 h the resulting silica was filtered off and washed 3 times with petroleum ether (30 ml). The silica was then transferred to a Soxhlet apparatus and extracted with diethyl ether for 16 h.
(b) Alumina obtained from (a) above (5g) was refluxed with pyridine (30 ml). After 16 h the resulting white solid was filtered off and washed with dichloromethane (3 x 30 ml) before Soxhlet extraction with diethylether for a further 16 h. The resulting alumina was dried in vacuo.
(c) [Rh(CO)₂Cl]₂ (0.02g) was dissolved in methanol (10 ml) and then added to the dried alumina from (b) above (1.8 g). The mixture was stirred for 5 minutes and the resulting brown alumina removed by filtration. The catalyst was found to contain 0.37% rhodium b.w. of dried material.

### Catalyst D (alternative (i))

The procedure used for the preparation of Catalyst B was followed except that the silica (FK 700) was replaced by gamma-alumina (CONDEA 200; 1.8 g).

### THE CARBONYLATION OF METHANOL

### Example 1

Catalyst A (2 ml) was loaded into a tubular reactor of 6-7 mm internal diameter and was tested in the carbonylation of a feedstock containing methanol and methyl iodide.

The feedstock was pumped to a preheated/vapouriser where it was evaporated and mixed with a flowing stream of carbon monoxide. The combined vapour/gas mixture was passed over the catalyst, and the outlet stream was analysed by gas chromatography at pre-determined time intervals, prior to cooling and condensation of the liquid products. The reactor was heated by a tubular furnace and the temperature measured by a thermocouple inserted into the catalyst bed. Pressure in the reactor was set and maintained by means of a batch pressure regulator. Liquid and gas feed rates to the vapouriser could be readily varied from 0-10 ml/h and 0-200 ml/m respectively.

The following conditions were established:

| | |
|---|---|
| LHSV | 1 |
| CO:MeOH:MeI molar ratio | 60:20:1 |
| Total Pressure | 9 barg |
| Temperature | 186°C |

The temperature was then held at 186°C for about 4 h before being increased. This procedure was repeated twice more before the temperature was reduced again to 186°C. Analysis of the product stream at hourly intervals then allowed the results presented in Table 1 to be calculated. With reference to Table 1, conversion is defined as the fraction (expressed as a percentage) of methanol converted to acetic acid, methyl acetate, dimethyl ether and methane, assuming that methyl acetate contains one converted and one unconverted methanol moiety (ie conversion does not take into account methanol converted by esterification).

**Table 1**

| Temperature °C | | Conversion * (%) | Product Proportions | | |
|---|---|---|---|---|---|
| Furnace | Reactor Bed | | MeOAc | AcOH | Me0 |
| 180 | 186 | 16.1 | 14.4 | 0.8 | 0.1 |
| 200 | 211 | 34.4 | 31.0 | 1.6 | 0.1 |
| 220 | 231 | 48.5 | 35.0 | 10.6 | 0.1 |
| 240 | 251 | 49.8 | 32.9 | 14.3 | 0.1 |
| 180 | 186 | 38.4 | 22.6 | 13.4 | 0.1 |

| | | | | | |
|---|---|---|---|---|---|
| *Major product was methyl acetate | | | | | |

### Comparison Test 1

Catalyst B (2 ml) was tested for the carbonylation of methanol at ca. 180°C and 9 barg using the procedure of Example 1. The conversion to carbonylated products was less than 1%.

This test demonstrates that rhodium supported on silica is not active as a catalyst and is included only for the purpose of comparison.

### Example 2

Catalyst C ( 2ml) was tested in the carbonylation of methanol at 181°C, 9 barg, LHSV = 1 and a CO:MeOH:MeI molar ratio = 60:20:1 using the experimental procedure outlined in Example 1. The duration of the experiment was 14½ h. Methanol conversions and product proportions after different times on stream are presented in Table 2.

**Table 2**

| Time on Stream (h) | Conversion (%) | Product Proportions | | |
|---|---|---|---|---|
| | | CH₃OCOCH₃ | CH₃CO₂H | CH₃OCH₃ |
| 4.5 | 10.2 | 8.5 | 1.5 | 0.1 |
| 6.5 | 9.5 | 8.1 | 1.4 | 0.1 |
| 8.5 | 9.8 | 8.4 | 1.2 | 0.1 |
| 10.5 | 9.6 | 8.6 | 0.9 | 0.1 |
| 12.5 | 9.3 | 8.2 | 1.0 | 0.1 |
| 14.5 | 9.6 | 8.6 | 0.8 | 0.1 |

### Comparison Test 2

Catalyst D (2 ml) was tested in the carbonylation of methanol according to the procedure of Example 1 under the following conditions: LHSV = 1, CO:MeOH:MeI molar ratio = 60:20:1, pressure = 9 barg and temperature = ca. 180°C. Conversion to carbonylation products was less than 1%, demonstrating that the alumina supported rhodium catalyst is not active under the conditions used.

This is not an example according to the present invention and is included for comparison purposes only.

## Claims

1. A process for the production from an alcohol having n carbon atoms, wherein n is a number from 1 to 20, of a carboxylic acid having n + 1 carbon atoms and/or an ester of the alcohol having n carbon atoms with the carboxylic acid by reacting the alcohol at a temperature of at least 50°C with carbon monoxide in the presence of a noble metal-containing heterogeneous catalyst and a halogen or halogen compound promoter
characterised in that the process comprises the steps of (a) vapourising the alcohol and (b) contacting the vapour of the alcohol with the carbon monoxide and the catalyst which comprises a noble metal component bonded to an inorganic oxide having surface hydroxyl groups through the intermediacy of a silicon compound which is condensible with the hydroxyl groups of the metal oxide and has either (i) at least one nitrogen containing functional group capable of coordinating with the noble metal component or (ii) a functional group capable of reacting further with a nitrogen
containing functional group capable of coordinating with the noble metal component thereby to bond the nitrogen containing functional group to the silicon compound.

2. A process according to claim 1 wherein the noble metal component is rhodium.

3. A process according to either one of the preceding claims wherein the inorganic oxide having surface hydroxyl groups is either silica or alumina.

4. A process according to any one of the preceding claims wherein the silicon compound which is condensible with the hydroxyl groups of the inorganic oxide contains a moiety -Y wherein -Y is either an -OR group in which R is an alkyl group, an ester group or a halide group.

5. A process according to claim 4 wherein -Y is an -OR group in which R is either methyl or ethyl.

6. A process according to either claim 4 or claim 5 wherein the silicon compound is one having a nitrogen containing functional group capable of coordinating with the noble metal component which functional group is capable of quaternisation.

7. A process according to any one of claims 1 to 3 wherein the silicon compound which is condensible with the hydroxyl groups of the inorganic oxide has at least one nitrogen containing functional group capable of coordinating with the noble metal component and has the formula: 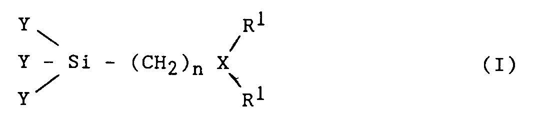 wherein the groups Y are independently -OR groups in which the groups R are either alkyl groups, ester groups or halide groups,
n is a number in the range from 1 to 6,
X is nitrogen, and
the groups R¹ are independently either hydrogen, alkyl or together form a ring which is either a carbocyclic or a heterocyclic nitrogen containing ring.

8. A process according to claim 7 wherein the silicon compound of formula (I) is either:
(A) 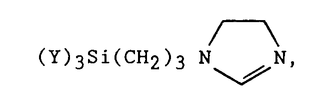
(B) 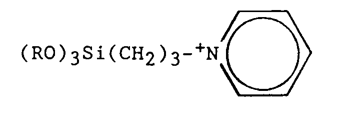 Cl⁻,
(C) (RO)₃Si(CH₂)₃NH₂.

9. A process according to claim 1 wherein the silicon compound which is condensible with the hydroxyl groups of the inorganic oxide has a functional group capable of reacting further with a nitrogen containing functional group capable of coordinating with the noble metal component thereby to bond the nitrogen containing functional group to the silicon compound.

10. A process according to any one of claims 1 to 8 wherein the catalyst is prepared by the steps comprising:
in a first step reacting an inorganic oxide having surface hydroxyl groups with a silicon compound having a first moiety which is condensible with hydroxyl groups and a second moiety which is a nitrogen containing functional group capable of coordinating a noble metal component under conditions whereby the hydroxyl groups of the inorganic oxide react with the first moiety thereby to bond the silicon compound to the inorganic oxide, and
in a second step reacting the second moiety of the product of the first step with a noble metal component, thereby to coordinate the noble metal component to the silicon compound.

11. A process according to claim 10 modified in that the noble metal component is reacted with the second moiety of the silicon compound before this latter compound is reacted via the first moiety with the inorganic oxide.

12. A process according to either claim 10 or claim 11 wherein the nitrogen containing functional group (the second moiety) is added in a pre-quaternised form.

13. A process according to any one of claims 1 to 8 wherein the catalyst is prepared by the steps comprising:-
in a first step reacting an inorganic oxide having surface hydroxyl groups with a silicon compound having a first moiety which is condensible with hydroxyl groups and a second moiety which is a nitrogen containing functional group capable of coordinating a noble metal component under conditions whereby the hydroxyl groups of the inorganic oxide react with the first moiety thereby to bond the silicon compound to the metal oxide,
in a second step reacting the product of the first step with a quaternising agent thereby to quaternise the nitrogen containing functional group, and
in a third step reacting the product of the second step with a noble metal halocarbonyl complex, thereby to form a noble metal halocarbonyl complex counter anion.

14. A process according to claim 13 wherein in the second step the quaternising agent is an alkyl iodide.

15. A process according to any one of claims 1 to 5 and 9 wherein the catalyst is prepared by the steps comprising:-
in a first step reacting an inorganic oxide having surface hydroxyl groups with a silicon compound having a first moiety which is condensible with hydroxyl groups and a second moiety which is a functional group capable of reacting further with a nitrogen containing compound capable of coordinating with a noble metal component under conditions whereby the hydroxyl groups of the inorganic oxide react with the first moiety thereby to bond the silicon compound to the inorganic oxide,
in a second step reacting the second moiety with a nitrogen containing compound under conditions whereby the nitrogen containing compound is bonded to the silicon compound, and
in a third step reacting the product of the second step with a noble metal component under conditions whereby the noble metal component is coordinatively bonded to the silicon compound.

16. A process according to any one of the preceding claims wherein the alcohol used as feedstock is an aliphatic alcohol having from 1 to 20 carbon atoms.

17. A process according to claim 16 wherein the alcohol is methanol and there is produced acetic acid and/or methyl acetate.

## Patentansprüche

1. Verfahren für die Herstellung einer Carbonsäure mit n + 1 Kohlenstoffatomen und/oder eines Esters des Alkohols mit n Kohlenstoffatomen mit der Carbonsäure aus einem Alkohol mit n Kohlenstoffatomen, worin n eine Zahl von 1 bis 20 ist, durch Umsetzen des Alkohols bei einer Temperatur von wenigstens 50°C mit Kohlenmonoxid in Anwesenheit eines edelmetallhaltigen heterogenen Katalysators und einem Halogen oder einer Halogenverbindung als Promotor, dadurch gekennzeichnet, daß das Verfahren die Schritte umfaßt (a) Verdampfen des Alkohols und (b) Kontaktieren des Alkoholdampfes mit dem Kohlenmonoxid und dem Katalysator, der einen Edelmetallbestandteil umfaßt, der an ein anorganisches Oxid mit Oberflächenhydroxylgruppen mittels einer Siliciumverbindung gebunden ist, die mit den Hydroxylgruppen des Metalloxids kondensierbar ist und entweder (i) wenigstens einen stickstoffhaltige funktionelle Gruppe, die mit dem Edelmetallbestandteil koordinieren kann oder (ii) eine funktionelle Gruppe, die weiter mit einer stickstoffhaltigen funktionellen Gruppe reagieren kann, die mit dem Edelmetallbestandteil koordinieren kann, aufweist, um dadurch die stickstoffhaltige funktionelle Gruppe an die Siliciumverbindung zu binden.

2. Verfahren nach Anspruch 1, worin der Edelmetallbestandteil Rhodium ist.

3. Verfahren nach einem der vorhergehenden Ansprüche, worin das anorganische Oxid mit Oberflächenhydroxylgruppen entweder Siliciumdioxid oder Aluminiumoxid ist.

4. Verfahren nach einem der vorhergehenden Ansprüche, worin die Siliciumverbindung, die mit den Hydroxylgruppen des anorganischen Oxids kondensierbar ist, einen -Y Teil enthält, worin -Y entweder eine -OR-Gruppe, worin R eine Alkylgruppe ist, eine Estergruppe oder eine Halogenidgruppe ist.

5. Verfahren nach Anspruch 4, worin -Y eine -OR Gruppe ist, worin R entweder Methyl oder Ethyl ist.

6. Verfahren nach Anspruch 4 oder 5, worin die Siliciumverbindung eine Verbindung mit einer stickstoffhaltigen funktionellen Gruppe ist, die mit dem Edelmetallbestandteil koordinieren kann, wobei die funktionelle Gruppe zur Quaternisierung fähig ist.

7. Verfahren nach einem der Ansprüche 1 bis 3, worin die Siliciumverbindung, die mit den Hydroxylgruppen des anorganischen Oxids kondensierbar ist, wenigstens eine stickstoffhaltige funktionelle Gruppe hat, die mit dem Edelmetallbestandteil koordinieren kann und die Formel hat: 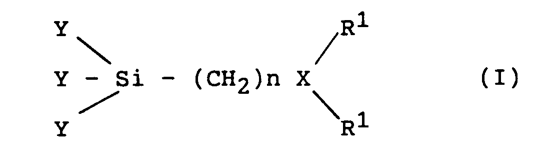 worin die Y-Gruppen unabhängig voneinander -OR-Gruppen sind, worin die R-Gruppen entweder Alkylgruppen, Estergruppen oder Halogenidgruppen sind,
n eine Zahl im Bereich von 1 bis 6 ist,
X Stickstoff ist, und
die R¹-Gruppen unabhängig entweder Wasserstoff oder Alkyl sind oder zusammen einen Ring bilden, der entweder ein carbozyklischer oder heterozyklischer, stickstoffhaltiger Ring ist.

8. Verfahren nach Anspruch 7, worin die Siliciumverbindung der Formel (I) entweder ist:
(A) 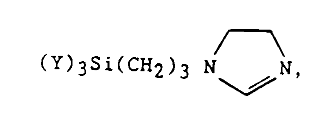
(B) 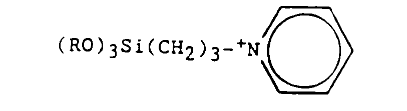 Cl⁻,
(C) (RO)₃Si(CH₂)₃NH₂.

9. Verfahren nach Anspruch 1, worin die Siliciumverbindung, die mit den Hydroxylgruppen des anorganischen Oxids kondensierbar ist, eine funktionelle Gruppe hat, die weiter mit einer stickstoffhaltigen funktionellen Gruppe reagieren kann, die mit dem Edelmetallbestandteil koordinieren kann, um dadurch die stickstoffhaltige funktionelle Gruppe an die Siliciumverbindung zu binden.

10. Verfahren nach einem der Ansprüche 1 bis 8, worin der Katalysator durch die folgenden Schritte hergestellt wird, die umfassen:
in einem ersten Schritt Umsetzen des anorganischen Oxids mit Oberflächenhydroxylgruppen mit einer Siliciumverbindung, die einen ersten Teil hat, der mit Hydroxylgruppen kondensierbar ist und einen zweiten Teil, der eine stickstoffhaltige funktionelle Gruppe ist, die einen Edelmetallbestandteil koordinieren kann unter Bedingungen, wobei die Hydroxylgruppen des anorganischen Oxids mit dem ersten Teil reagieren, um dadurch die Siliciumverbindung an das anorganische Oxid zu binden, und
in einem zweiten Schritt Umsetzen des zweiten Teils des Produkts aus dem ersten Schritt mit einem Edelmetallbestandteil, um dadurch den Edelmetallbestandteil an die Siliciumverbindung zu koordinieren.

11. Verfahren nach Anspruch 10, dadurch modifiziert, daß der Edelmetallbestandteil mit dem zweiten Teil der Siliciumverbindung umgesetzt wird, bevor diese letztere Verbindung über den ersten Teil mit dem anorganischen Oxid umgesetzt wird.

12. Verfahren nach Anspruch 10 oder 11, worin die stickstoffhaltige funktionelle Gruppe (der zweite Teil) in präquaternisierter Form zugegeben wird.

13. Verfahren nach einem der Ansprüche 1 bis 8, worin der Katalysator durch die Schritte hergestellt wird, umfassend:
in einem ersten Schritt Umsetzen des anorganischen Oxids mit Oberflächenhydroxylgruppen mit einer Siliciumverbindung, die einen ersten Teil hat, der mit Hydroxylgruppen kondensierbar ist und einen zweiten Teil, der eine stickstoffhaltige funktionelle Gruppe ist, die einen Edelmetallbestandteil koordinieren kann unter Bedingungen, wobei die Hydroxylgruppen des anorganischen Oxids mit dem ersten Teil reagieren, um dadurch die Siliciumverbindung an das Metalloxid zu binden.
in einem zweiten Schritt Umsetzen des Produkts aus dem ersten Schritt mit einem Quaternisierungsmittel, um dadurch die stickstoffhaltige funktionelle Gruppe zu quaternisieren und
in einem dritten Schritt Umsetzen des Produkts aus dem zweiten Schritt mit einem Edelmetallhalocarbonylkomplex, um dadurch ein Edelmetallhalocarbonylkomplex-Gegenanion zu bilden.

14. Verfahren nach Anspruch 13, worin im zweiten Schritt das Quaternisierungsmittel ein Alkyljodid ist.

15. Verfahren nach einem der Ansprüche 1 bis 5 und 9, worin der Katalysator durch die Schritte hergestellt wird, umfassend:-
in einem ersten Schritt Umsetzen des anorganischen Oxids mit Oberflächenhydroxylgruppen mit einer Siliciumverbindung, die einen ersten Teil hat, der mit Hydroxylgruppen kondensierbar ist und einen zweiten Teil, der eine funktionelle Gruppe ist, die weiter mit einer stickstoffhaltigen Verbindung reagieren kann, die mit einem Edelmetallbestandteil koordinieren kann unter Bedingungen, wobei die Hydroxylgruppen des anorganischen Oxids mit dem ersten Teil reagieren, um dadurch die Siliciumverbindung an das anorganische Oxid zu binden,
in einem zweiten Schritt Umsetzen des zweiten Teils mit einer stickstoffhaltigen Verbindung unter Bedingungen, wobei die stickstoffhaltige Verbindung an die Siliciumverbindung gebunden wird, und
in einem dritten Schritt Umsetzen des Produkts aus dem zweiten Schritt mit einem Edelmetallbestandteil unter Bedingungen, wobei der Edelmetallbestandteil koordinativ an die Siliciumverbindung gebunden wird.

16. Verfahren nach einem der vorhergehenden Ansprüche, worin der als Ausgangsmaterial verwendete Alkohol ein aliphatischer Alkohol mit 1 bis 20 Kohlenstoffatomen ist.

17. Verfahren nach Anspruch 16, worin der Alkohol Methanol ist und Essigsäure und/oder Methylacetat hergestellt wird.

## Revendications

1. Procédé de production, à partir d'un alcool ayant n atomes de carbone, où n est un nombre de 1 à 20, d'un acide carboxylique ayant n + 1 atomes de carbone et/ou d'un ester de l'alcool ayant n atomes de carbone avec l'acide carboxylique par réaction de l'alcool à une température d'au moins 50°C avec l'oxyde de carbone en présence d'un catalyseur hétérogène contenant un métal noble et d'un activateur formé d'un halogène ou d'un composé d'halogène, caractérisé en ce qu'il comprend les étapes consistant (a) à vaporiser l'alcool et (b) à faire entrer la vapeur de l'alcool en contact avec l'oxyde de carbone et le catalyseur qui comprend un composant à base d'un métal noble lié à un oxyde inorganique portant des groupes hydroxyle en surface, par l'intermédiaire d'un composé de silicium qui peut être condensé avec les groupes hydroxyle de l'oxyde métallique et a (i) au moins un groupe fonctionnel contenant de l'azote capable de coordination avec le composant à base de métal noble ou (ii) un groupe fonctionnel capable de réagir encore avec un groupe fonctionnel contenant de l'azote capable de coordination avec le composant à base de métal noble de manière à lier ainsi le groupe fonctionnel contenant de l'azote au composé de silicium.

2. Procédé suivant la revendication 1, dans lequel le composant à base d'un métal noble est le rhodium.

3. Procédé suivant l'une des revendications précédentes, dans lequel l'oxyde inorganique portant des groupes hydroxyle en surface est la silice ou l'alumine.

4. Procédé suivant l'une quelconque des revendications précédentes, dans lequel le composé de silicium qui peut être condensé avec les groupes hydroxyle de l'oxyde inorganique contient un groupement -Y qui est un groupe -OR dans lequel R est un groupe alkyle, un groupe ester ou un groupe halogénure.

5. Procédé suivant la revendication 4, dans lequel -Y est un groupe -OR dans lequel R est un groupe méthyle ou éthyle.

6. Procédé suivant la revendication 4 ou la revendication 5, dans lequel le composé de silicium est un composé portant un groupe fonctionnel contenant de l'azote capable de coordination avec le composant à base de métal noble, ce groupe fonctionnel étant capable de quaternisation.

7. Procédé suivant l'une quelconque des revendications 1 à 3, dans lequel le composé de silicium qui peut être condensé avec les groupes hydroxyle de l'oxyde inorganique porte au moins un groupe fonctionnel contenant de l'azote capable de coordination avec le composant à base d'un métal noble et répond à la formule : 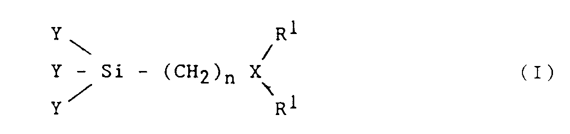 dans laquelle les groupes Y sont, indépendamment, des groupes -OR dans lesquels les groupes R sont des groupes alkyle, des groupes ester ou des groupes halogénure, n est un nombre compris dans la plage de 1 à 6, X est l'azote, et les groupes R¹ sont, indépendamment, de l'hydrogène, des groupes alkyle, ou forment conjointement un noyau qui est un noyau carbocyclique ou un noyau hétérocyclique contenant de l'azote.

8. Procédé suivant la revendication 7, dans lequel le composé de silicium de formule (I) est l'un des composés :
(A) 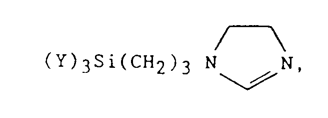
(B) 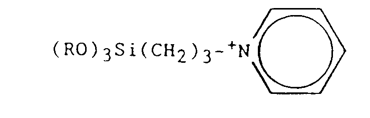 Cl⁻,
(C) (RO)₃Si(CN₂)₃NH₂.

9. Procédé suivant la revendication 1, dans lequel le composé de silicium qui peut être condensé avec les groupes hydroxyle de l'oxyde inorganique porte un groupe fonctionnel capable de réagir encore avec un groupe fonctionnel contenant de l'azote capable de coordination avec le composant à base d'un métal noble, de manière à lier ainsi le groupe fonctionnel contenant de l'azote au composé de silicium.

10. Procédé suivant l'une quelconque des revendications 1 à 8, dans lequel le catalyseur est préparé par les étapes consistant :
à faire réagir, dans une première étape, un oxyde inorganique portant des groupes hydroxyle en surface avec un composé de silicium ayant un premier groupement qui peut être condensé avec des groupes hydroxyle et un second groupement qui est un groupe fonctionnel contenant de l'azote capable de coordonner un composant à base de métal noble dans des conditions dans lesquelles les groupes hydroxyle de l'oxyde inorganique réagissent avec le premier groupement pour lier ainsi le composé de silicium à l'oxyde inorganique, et
à faire réagir, dans une seconde étape, le second groupement du produit de la première étape avec un composant à base d'un métal noble, de manière à coordonner ainsi le composant à base d'un métal noble au composé de silicium.

11. Procédé suivant la revendication 10, modifié en ce sens que le composant à base de métal noble est amené à réagir avec le second groupement du composé de silicium avant que ce dernier composé ne réagisse par l'intermédiaire du premier groupement avec l'oxyde inorganique.

12. Procédé suivant la revendication 10 ou la revendication 11, dans lequel le groupe fonctionnel contenant de l'azote (second groupement) est ajouté sous une forme préquaternisée.

13. Procédé suivant l'une quelconque des revendications 1 à 8, dans lequel le catalyseur est préparé par les étapes consistant :
à faire réagir, dans une première étape, un oxyde inorganique portant des groupes hydroxyle en surface avec un composé de silicium portant un premier groupement qui peut être condensé avec des groupes hydroxyle et un second groupement qui est un groupe fonctionnel contenant de l'azote, capable de coordiner un composant à base de métal noble dans des conditions dans lesquelles les groupes hydroxyle de l'oxyde inorganique réagissent avec le premier groupement afin de lier ainsi le composé de silicium à l'oxyde métallique,
à faire réagir, dans une deuxième étape, le produit de la première étape avec un agent de quaternisation de manière à quaterniser ainsi le groupe fonctionnel contenant de l'azote, et
à faire réagir, dans une troisième étape, le produit de la deuxième étape avec un complexe halogénocarbonylique de métal noble de manière à former ainsi un anion complémentaire du complexe halogénocarbonylique de métal noble.

14. Procédé suivant la revendication 13, dans la deuxième étape duquel l'agent de quaternisation est un iodure d'alkyle.

15. Procédé suivant l'une quelconque des revendications 1 à 5 et 9, dans lequel le catalyseur est préparé par les étapes consistant :
à faire réagir, dans une première étape, un oxyde inorganique portant des groupes hydroxyle en surface avec un composé de silicium ayant un premier groupement qui peut être condensé avec des groupes hydroxyle et un second groupement qui est un groupe fonctionnel capable de réagir encore avec un composé contenant de l'azote capable de coordination avec un composant à base de métal noble dans des conditions dans lesquelles les groupes hydroxyle de l'oxyde inorganique réagissent avec le premier groupement de manière à lier ainsi le composé de silicium à l'oxyde inorganique,
à faire réagir, dans une seconde étape, le second groupement avec un composé contenant de l'azote dans des conditions dans lesquelles le composé contenant de l'azote est lié au composé de silicium, et
à faire réagir, dans une troisième étape, le produit de la deuxième étape avec un composant à base de métal noble dans des conditions dans lesquelles le composant à base de métal noble est lié par coordination au composé de silicium.

16. Procédé suivant l'une quelconque des revendications précédentes, dans lequel l'alcool utilisé comme charge est un alcool aliphatique ayant 1 à 20 atomes de carbone.

17. Procédé suivant la revendication 16, dans lequel l'alcool est le méthanol et le produit formé est l'acide acétique et/ou l'acétate de méthyle.
